# EUROPEAN PATENT SPECIFICATION

(11) **EP 0 193 586 B1**
(45) Date of publication and mention of the grant of the patent: **04.08.1993**
(21) Application number: 85904583.3
(22) Date of filing: 10.09.1985
(51) Int. Cl.: C07H 21/04, C12N 15/00, C12N 1/20, C12P 19/34, C12P 21/02, C07K 13/00, C07G 17/00, A61K 39/015, G01N 33/53

(54) **ANTIGENS OF $i(PLASMODIUM FALCIPARUM)**
ANTIGENE AUS PLASMODIUM-FALCIPARUM
ANTIGENES DE $i(PLASMODIUM FALCIPARUM)

(30) Priority: 11.09.1984 AU 7066/84; 11.09.1984 AU 7067/84
(43) Date of publication of application: 10.09.1986
(73) Proprietor: SARAMANE PTY. LTD., Kooyong, VIC 3144 (AU)
(72) Inventor: KEMP, David, James, North Balwyn, VIC 3103 (AU); ANDERS, Robin, Fredric, North Melbourne, VIC 3051 (AU); COPPEL, Ross, Leon, Armadale, VIC 3143 (AU); BROWN, Graham, Vallancey, Balwyn, VIC 3103 (AU); SAINT, Robert, Bryce, Lower Templestowe, VIC 3107 (AU); COWMAN, Alan, Frederick, North Carlton, VIC 3054 (AU); BIANCO, Albert, Edward, Ascot Vale, VIC 3032 (AU); MITCHELL, Graham, Frank, Lower Templestowe, VIC 3107 (AU)
(74) Representative: Holmes, Michael John
(86) International application number: AU8500223
(87) International publication number: WO8601802

(56) References cited:
- WO-A-84/02917
- AU-A- 2 384 284
- AU-A- 3 904 685
- NATURE, vol. 310, 30 August 1984; R.L. COPPEL et al., pp. 789-792#
- PROCEEDINGS OF THE NATL. ACADEMY OF SCIENCES USA, vol. 82, January 1985; H.-D. STAHL et al., pp. 543-547#

## Description

This invention relates to synthetic peptides and polypeptides which have antigenicity suitable for providing protective immunity against Plasmodium falciparum infections, and to methods for the production thereof.

Immunity to Plasmodium falciparum, the protozoan causing the most severe form of human malaria, is acquired only after extensive exposure over a number of years. A large number of P.falciparum polypeptides are natural immunogens in man but it is by no means clear how many are important in protective immunity. Many antigens may have no such role, and indeed it is possible that some are counterproductive, perhaps because collectively they overload the immune system. Antigenic diversity among different strains of the parasite may also play a significant role in the process of immune evasion as a number of P.falciparum antigens that are strain-specific have been identified.

Recently, molecular cloning techniques have facilitated the analysis of individual polypeptide antigens of P.falciparum (1). Many cDNA clones encoding these antigens have been isolated by screening Escherichia coli colonies that express the cloned sequences with human antibodies. The production and screening of these clones is described in detail in International Patent Specification No. PCT/AU84/00016.

One such antigen has been located at the surface of erythrocytes infected with the immature ring stage of P.falciparum and hence has been designated the Ring-infected Erythrocyte Surface Antigen (RESA). Because of this exposed location, it appears to be a likely target for immune attack. RESA shows the structural peculiarity that has now been found in a number of Plasmodium antigens, namely multiple tandem repeats of oligopeptides (2-6).

Studies by hybridization and by immunofluorescence suggest that RESA from the Papua New Guinea isolate FC27, may be conserved in a wide range of P.falciparum isolates, including strain NF7 from Ghana. The relationship between RESA cDNA clones from two different strains of P.falciparum has therefore been studied by immunological and sequencing methods. Antibodies that reacted with RESA from strain FC27 of Papua New Guinea were present in patients from Africa and conversely, antibodies that reacted with RESA from strain NF7 were present in patients from Papua New Guinea. From the complete nucleotide sequences of eight cDNA clones encoding portions of RESA from P.falciparum strains FC27 and NF7, it is concluded that the RESA polypeptides from the two strains are closely homologous. The sequencing of these cDNA clones identified in the RESA polypeptide two separate blocks of tandem sequence repeats. One block of repeats, located at the C terminus of RESA in FC27, contains four different but related acidic sequences of eight, four, four and three amino acids. Approximately 600 bases 5' is a second block of repeats encoding related amino acid sequences which are also rich in acidic amino acids. Consistent with the sequence relationships, the two blocks of repeats have been shown to encode cross-reacting antigenic epitopes.

Immunoblots on the antigens of synchronously growing parasites separated on SDS-PAGE suggested that RESA is synthesized in the mature trophozoite as a Mr 210,000 protein which is processed to the Mr 155,000 form found bound to the membrane of erythrocytes infected with ring stage parasites. The more recent finding that the Mr 210,000 protein does not react with several anti-RESA monoclonal antibodies and anti-RESA peptide antibodies suggests that the Mr 210,000 protein is a cross-reacting antigen and not a precursor of the Mr 155,000 RESA molecule.

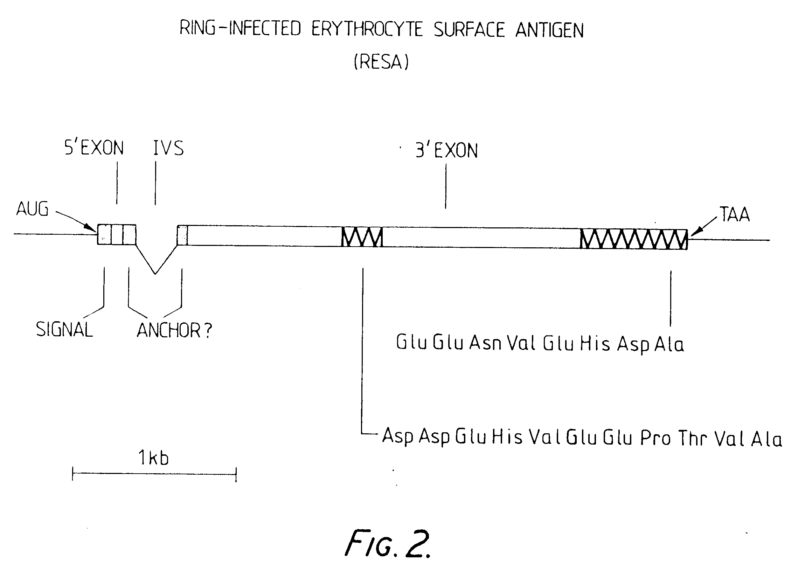

The Mr 155,000 polypeptide in merozoites is soluble in the non-ionic detergent Triton X-100 but after transfer to the membrane of the ring-infected erythrocyte it is largely Triton-insoluble. Thus, it seems likely that RESA interacts with the erythrocyte cytoskeleton. Whether RESA penetrates the membrane lipid bilayer is not yet clear, but an important clue may come from the complete sequence of the RESA gene which has now been determined. From this, it is deduced that RESA contains two exons separated by a short intervening sequence (Figure 2). Exon 1 commences with a hydrophobic sequence typical of signal peptides on secreted polypeptides in many organisms. Following this, there is a hydrophilic sequence of approximately 36 amino acid residues and then a second hydrophobic stretch, of 14 residues. 202 bases further downstream exon 2 commences with a 16 amino acid non-charged sequence and then continues with a highly charged region. The hydrophobic sequence generated by excision of the intron is typical of membrane-anchor segments in a number of eukaryotic genes.

As a result of work leading to the present invention, described in detail below, it has been shown on the basis of sequence, hybridization and immunological data that it is likely that RESA is highly conserved in most or all strains of P.falciparum. In addition, as the repetitive structure and the location of RESA at the surface of ring infected erythrocytes are properties highly suited for sensitive detection by such procedures as indirect immunofluorescence, the high degree of immunological similarity of RESA in different strains suggest that RESA is a molecule well suited for immunodiagnostic purposes.

Another antigen detected as a result of its cloning and expression in E.coli has been designated the Falciparum Interspersed Repeat Antigen (FIRA) (6). Like some other repetitive antigens FIRA contains a structural unit bearing repeats of a short unit flanked by a highly charged region. However, this entire structural unit is itself repeated several times within the antigen.

The corresponding cDNA clone expressing FIRA in Escherichia coli reacted in an in situ colony assay with sera from up to ∼93% of people living in an area endemic for P.falciparum. Human antibodies affinity-purified on immobilized lysates of the corresponding cDNA clone identified the corresponding parasite antigen as a polypeptide of Mr >300,000. It was present in schizonts and also in ring-stage trophozoites, where a speckled immunofluorescence pattern suggested an association with the erythrocyte. Its mRNA was enriched in merozoites, a distinctive property shared by RESA which is located on the surface of ring-infected erythrocytes and it is encoded by a single gene with a number of allelic variants. The complete nucleotide sequence of the cDNA clone revealed a structural unit comprised of 13 hexapeptide repeats flanked by a highly charged region containing both acidic and basic amino acids. This structural unit is itself repeated, so that blocks of repeats and charged units are interspersed along the molecule. The sequence within the repeats vary much more extensively than those in the charged units.

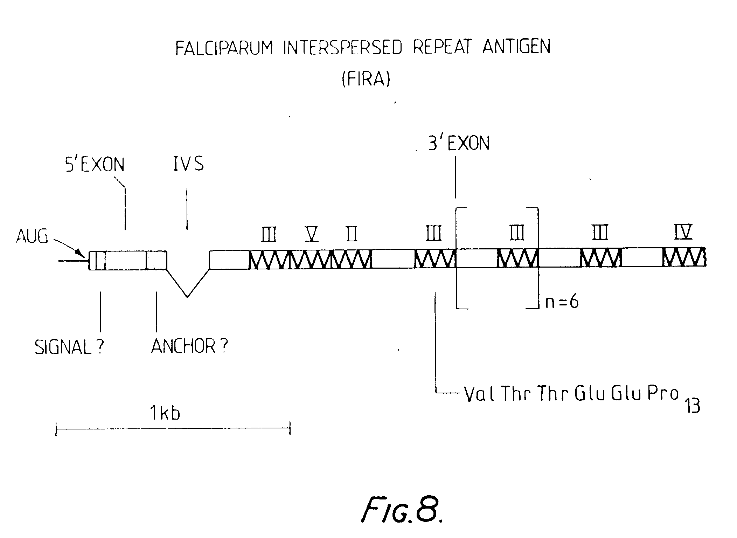

The sequence of a chromosomal FIRA clone demonstrates that the FIRA gene is organised in a manner analogous to that of RESA (Figure 8). It contains a short 5' exon, a much longer 3' exon and a hydrophobic segment at the boundary of the two exons. As with RESA, the repeats in FIRA are restricted to the 3' exon only.

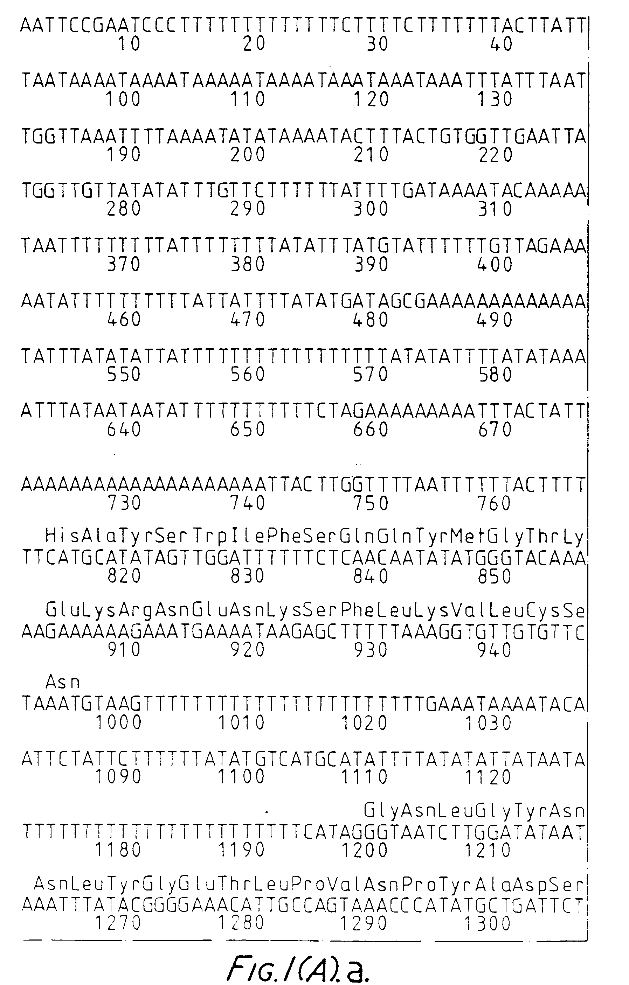

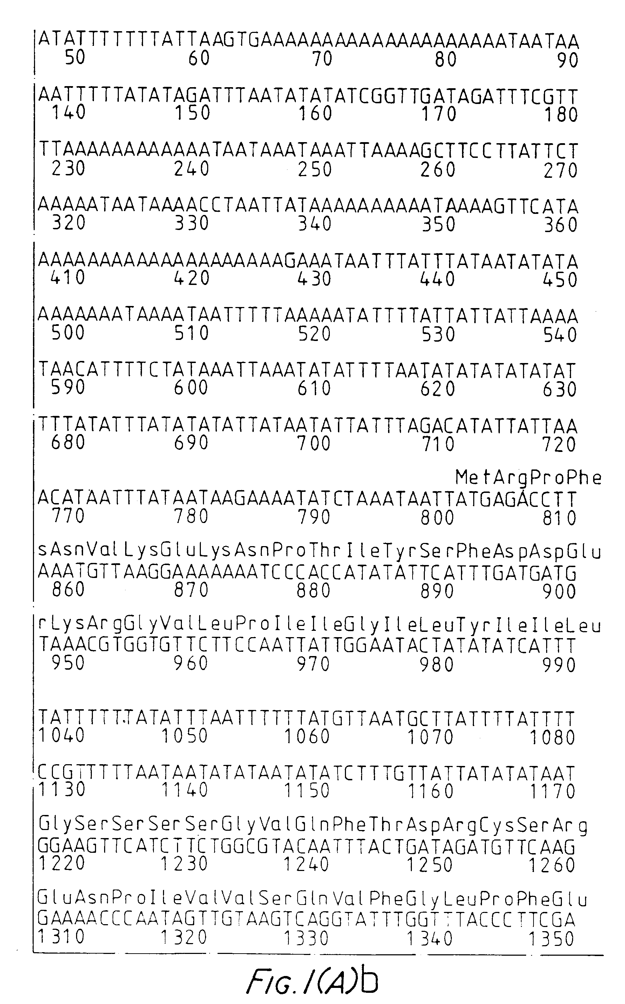

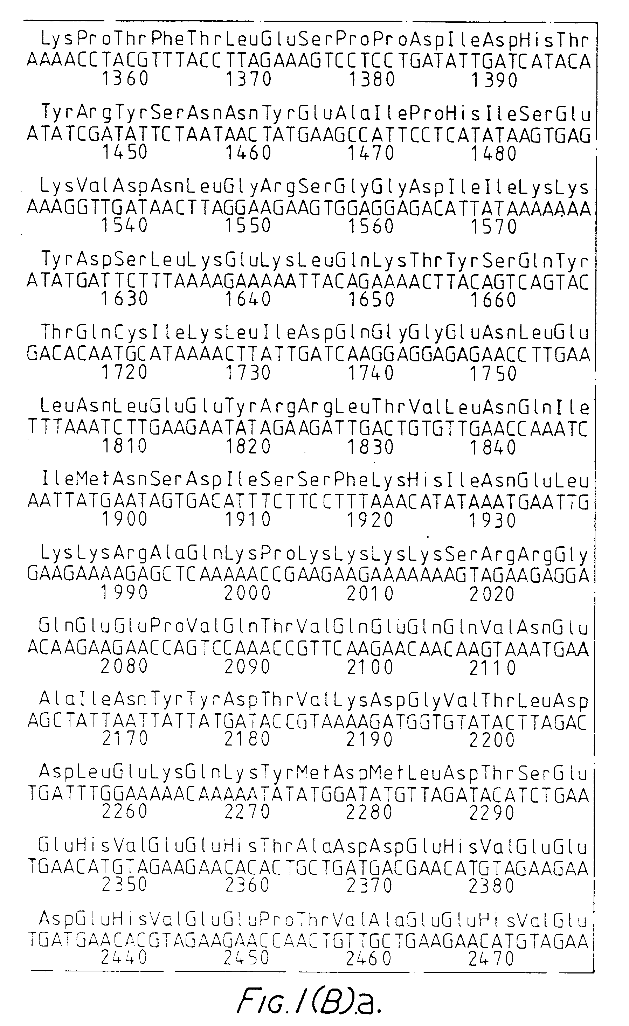

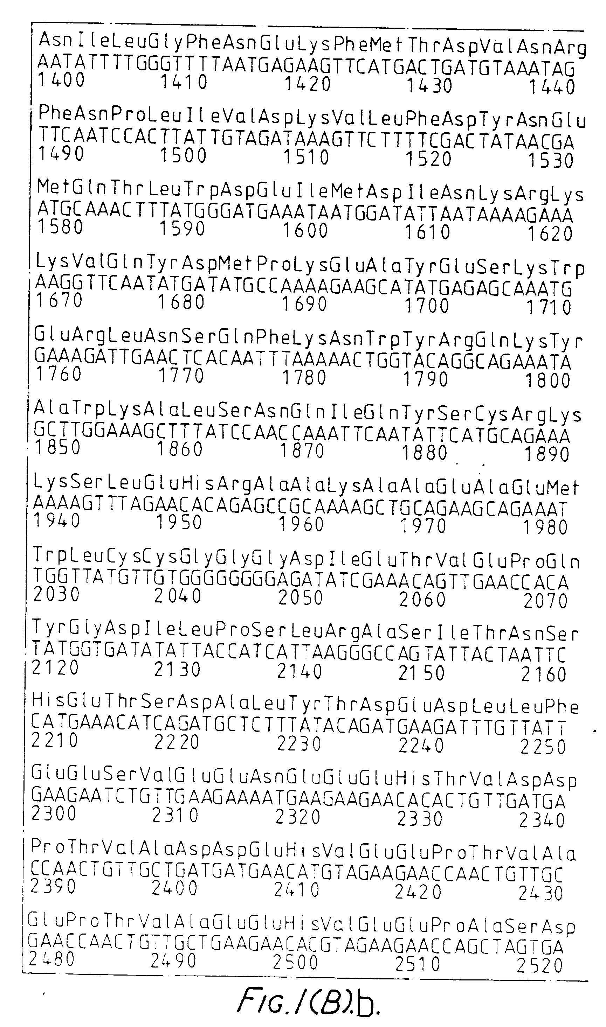

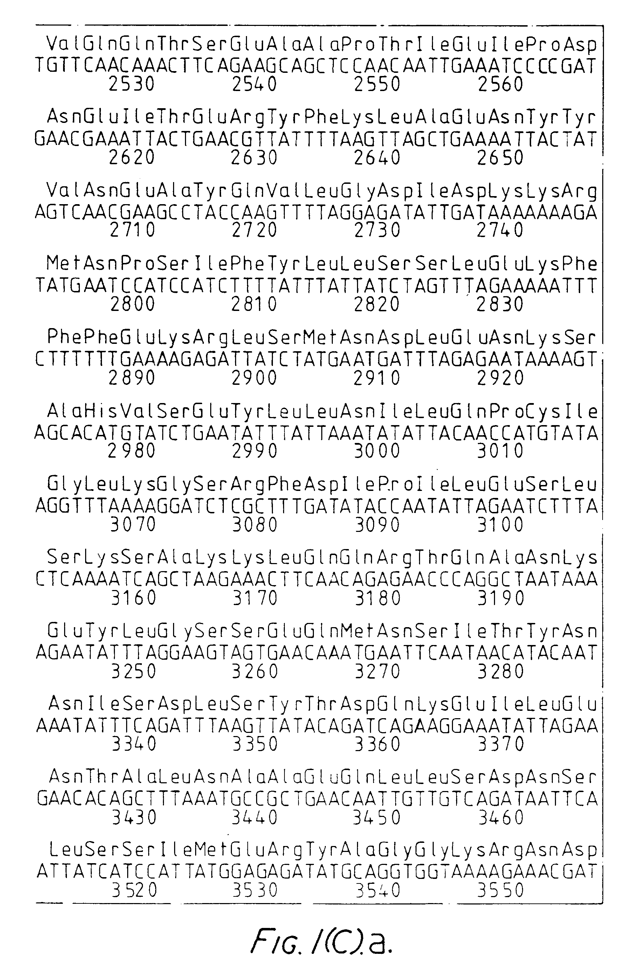

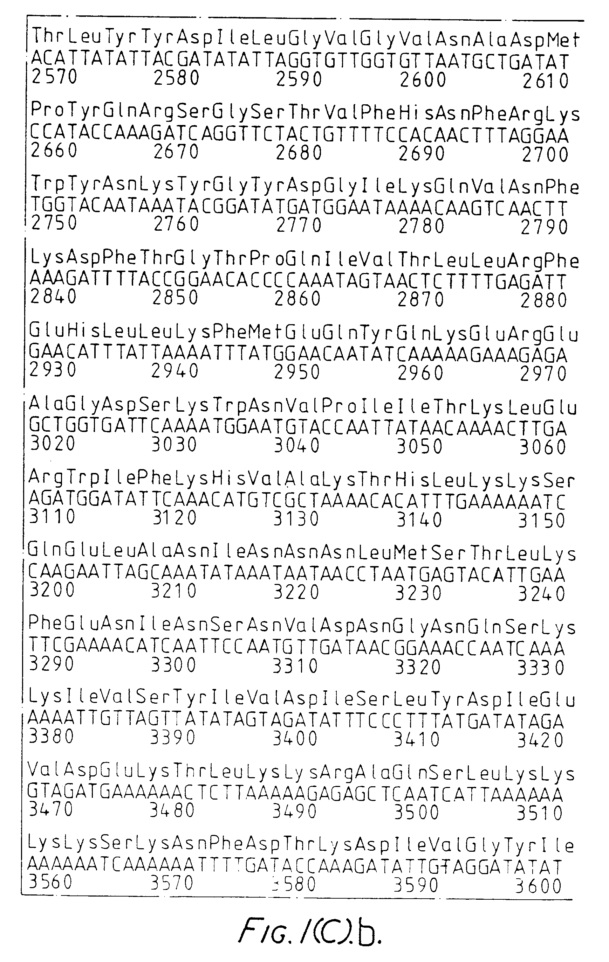

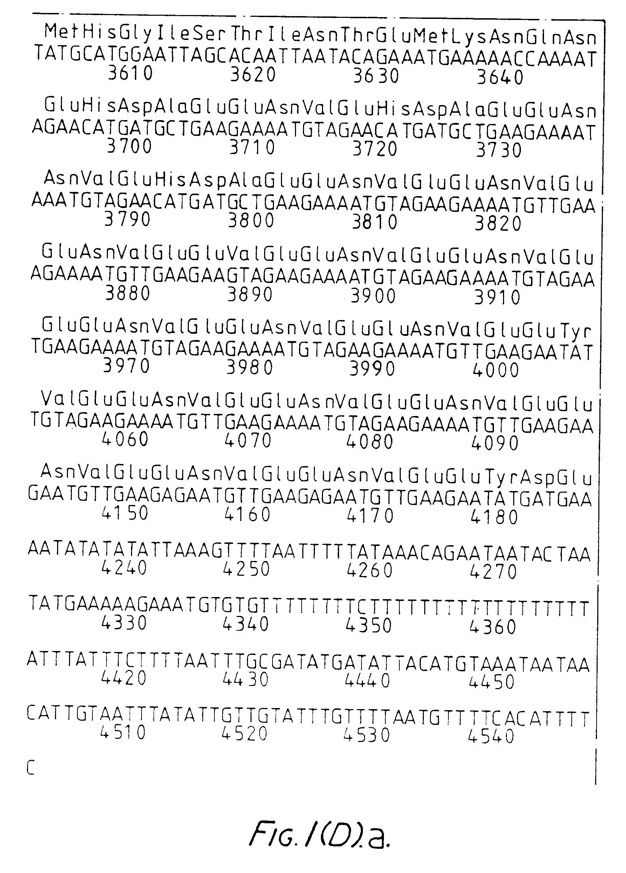

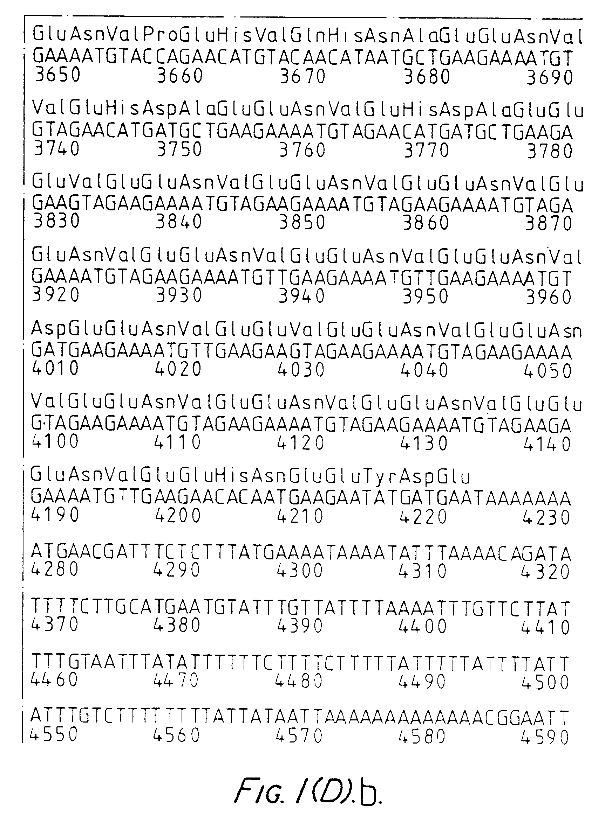

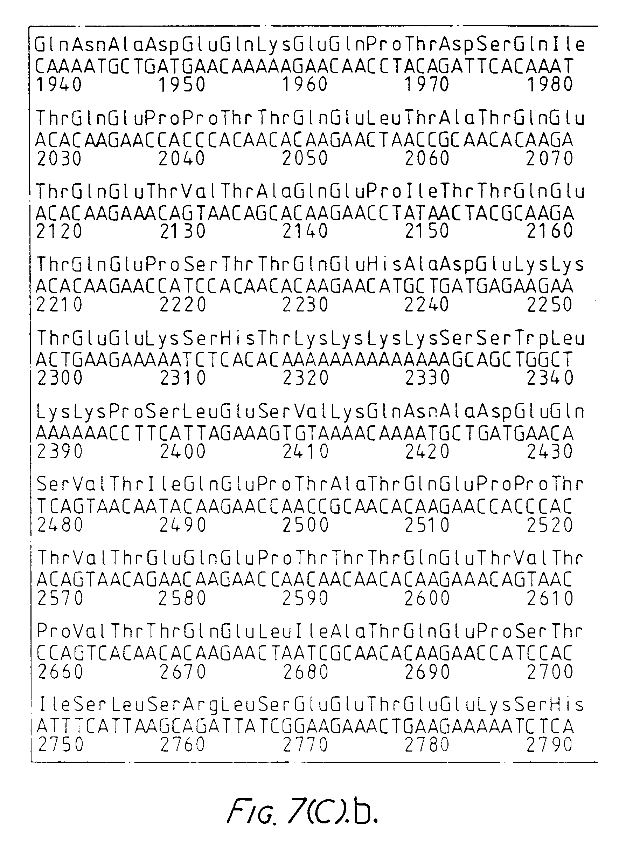

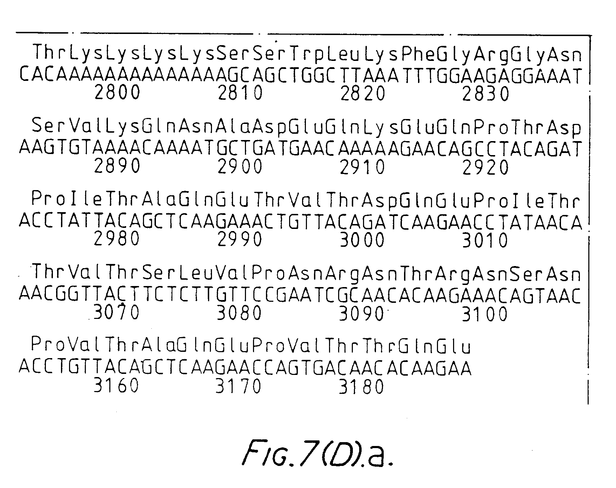

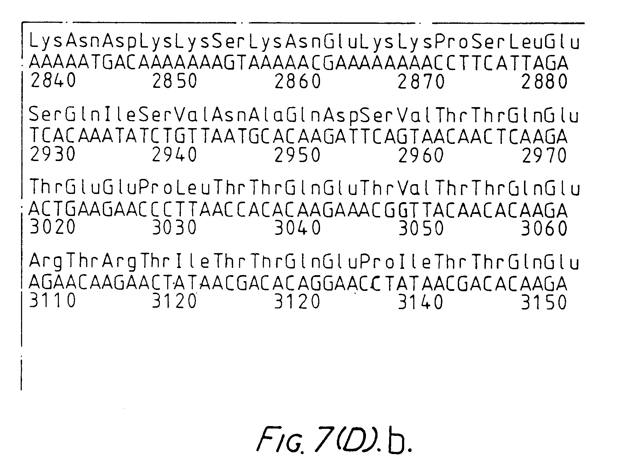

According to the present invention, there is provided a DNA molecule comprising a nucleotide sequence coding for an immunogenic polypeptide of P.falciparum, characterised in that said nucleotide sequence substantially corresponds to all or a portion of the base sequence as shown in Figure 7 coding for the Falciparum Interspersed Repeat Antigen (FIRA); or substantially corresponds to all of the base sequence as shown in Figure 1 coding for the Ring-Infected Erythrocyte Surface Antigen (RESA) or a portion thereof other than a portion consisting solely of the sequence 3619-3996 of Figure 1 or portions thereof; or a functional equivalent of any of the foregoing nucleotide sequences.

As noted above, and set out in greater detail in Figure 1 and 7, the amino acid sequences of RESA and FIRA consist of repeat units and flanking non-repeat peptide units. Accordingly, the base sequences referred to above may code for polypeptides corresponding to one or more of these repeat and/or flanking units, or to polypeptides corresponding to combinations of these repeat and/or flanking units.

The present invention also extends to synthetic peptides or polypeptides comprising an amino acid sequence displaying the antigenicity of all or a portion of an immunogenic polypeptide of P.falciparum, characterised in that said amino acid sequence substantially corresponds to the amino acid sequence of the FIRA antigen as shown in Figure 7 or an immunogenic portion thereof; or substantially corresponds to the amino acid sequence of the RESA antigen as shown in Figure 1 or an immunogenic portion thereof other than a portion encoded by the sequence 3619-3996 of Figure 1 or portions thereof; or to a functional equivalent of any of the foregoing amino acid sequences, as well as to compositions for stimulating immune responses against P.falciparum in a mammal, which compositions comprise at least one synthetic peptide or polypeptide as described above, together with a pharmaceutically acceptable carrier therefor. The synthetic peptides or polypeptides according to this aspect of the invention may be prepared by expression in a host cell containing a recombinant DNA molecule which comprises a nucleotide sequence as broadly described above operatively linked to an expression control sequence, or a recombinant DNA cloning vehicle or vector containing such a recombinant DNA molecule. The synthetic peptide or polypeptide so expressed may be a fusion polypeptide comprising a portion displaying the antigenicity of all or a portion of RESA or FIRA or other cross-reactive antigen, and an additional polypeptide coded for by the DNA of the recombinant DNA molecule fused thereto. Alternatively, the synthetic peptides or polypeptides may be produced by chemical means, such as by the well-known Merrifield solid-phase synthesis procedure.

Further details of the present invention will be apparent from the detailed description hereunder, and from the accompanying Figures. In the Figures:
- Figure 1: shows the nucleotide sequence and predicted amino acid sequence of RESA. The nucleotide sequence was determined by the dideoxy procedure (8).
- Figure 2: shows the structure of the RESA gene, as deduced from the sequence given in Figure 1. The 5' and 3' exons are indicated.
- Figure 3: shows:
A. Western blot of asynchronous cultures of two isolates of P.falciparum lysed in electrophoresis sample buffer and probed with anti-RESA antibodies.
B.& C. Western blots of P.falciparum (1) ring stages, (2) mature trophozoites, (3) schizonts, and (4) merozoites using affinity-purified human antibodies to RESA. (B) Antigens extracted in Triton X-100. (C) Antigens insoluble in Triton X-100 but soluble in electrophoresis sample buffer. Radioactive molecular weight markers were obtained from Amersham Internat., Buckinghamshire, England and were myosin (200 Kdaltons), phosphorylase-b (93 Kdaltons) and bovine serum albumin (69 Kdaltons).

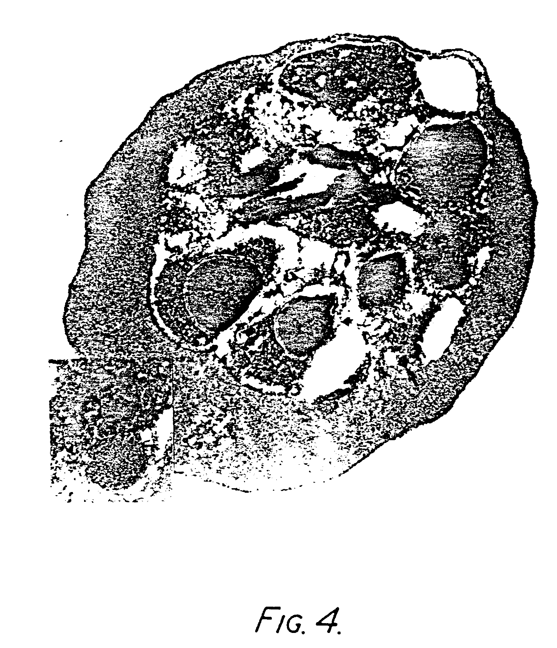

- Figure 4: is an immunoelectronmicrograph showing the location of RESA (→) in small dense vesicles presumably micronemes within the developing merozoites in a schizont, detected with rabbit anti-RESA and protein A gold. The rhoptries (R) are unlabelled. (x 41,700; inset x 73,000).

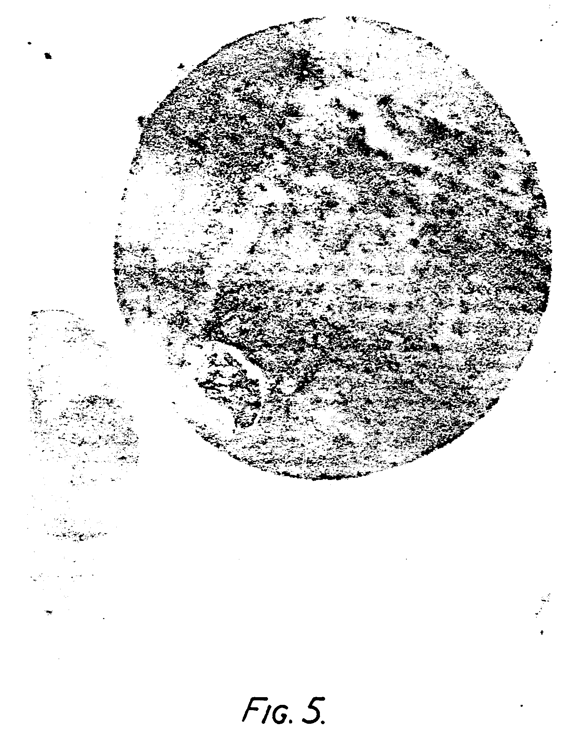

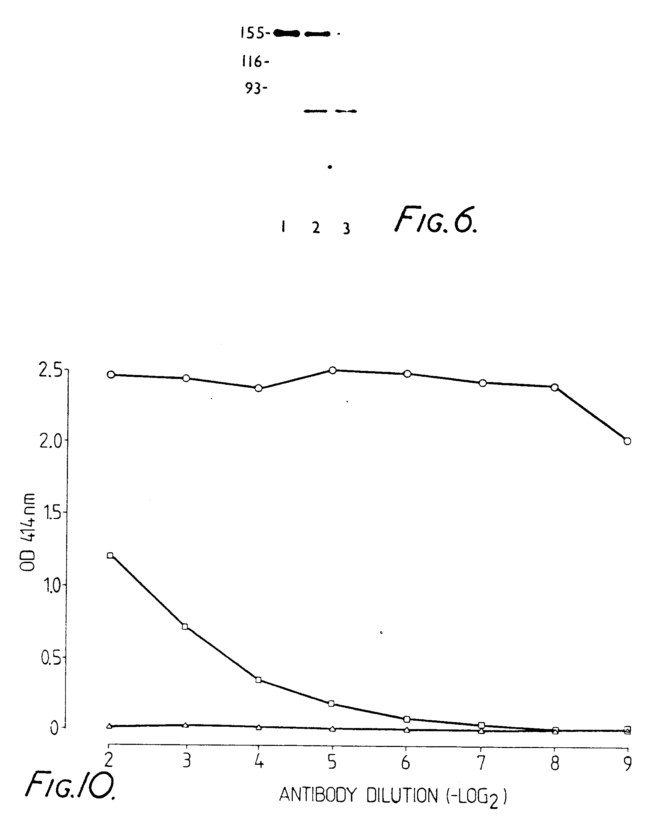

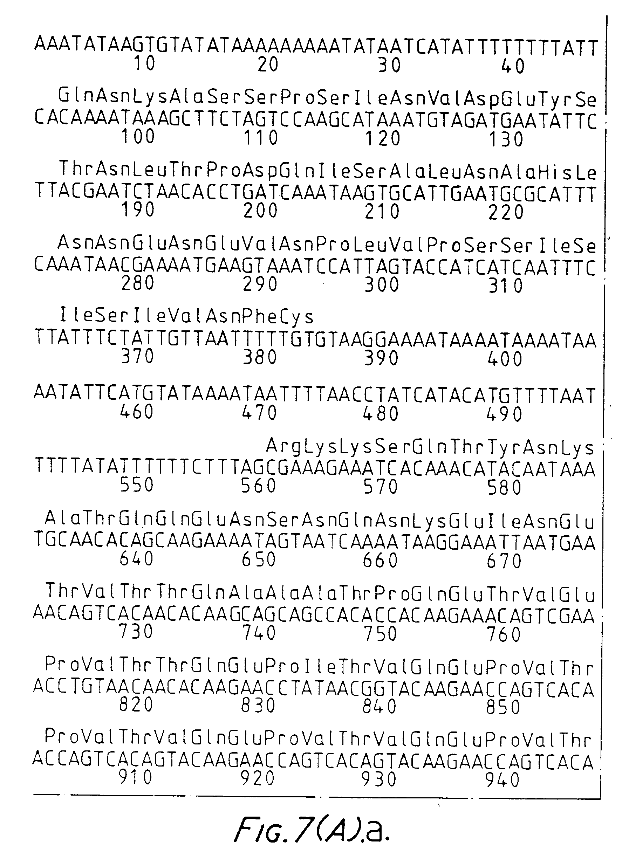

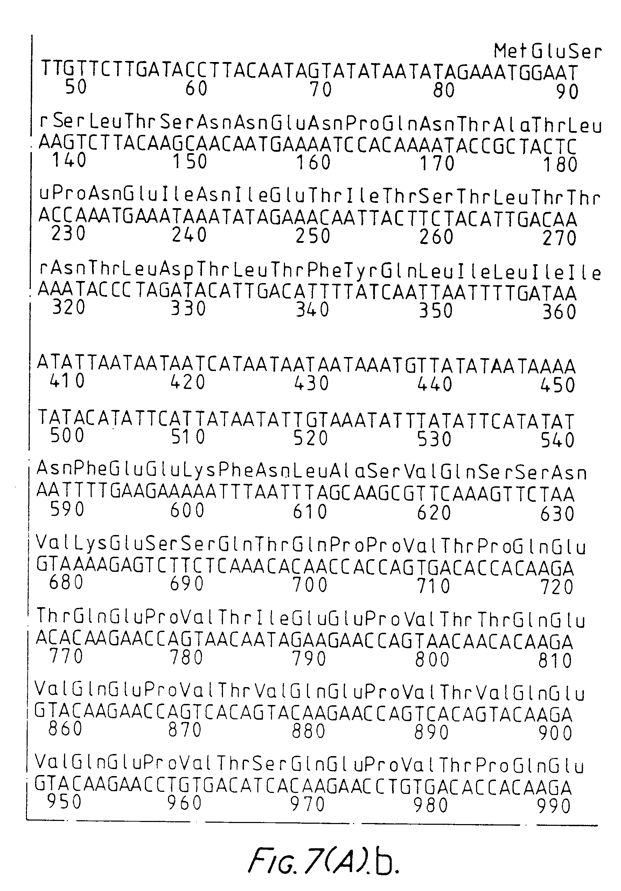

- Figure 5: is an immunoelectronmicrograph showing a section of a ring-infected erythrocyte reacted with rabbit anti-RESA. Also shown is part of an uninfected erythrocyte.

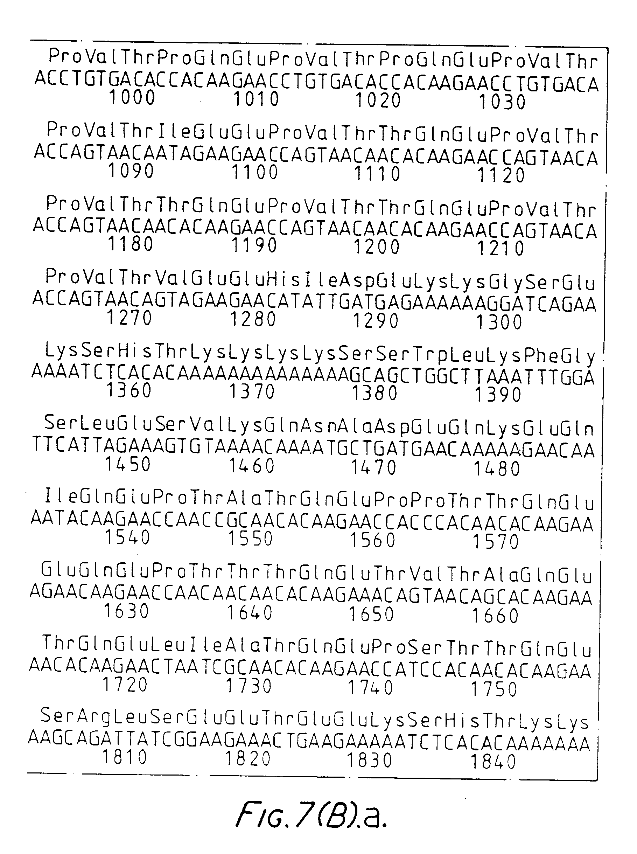

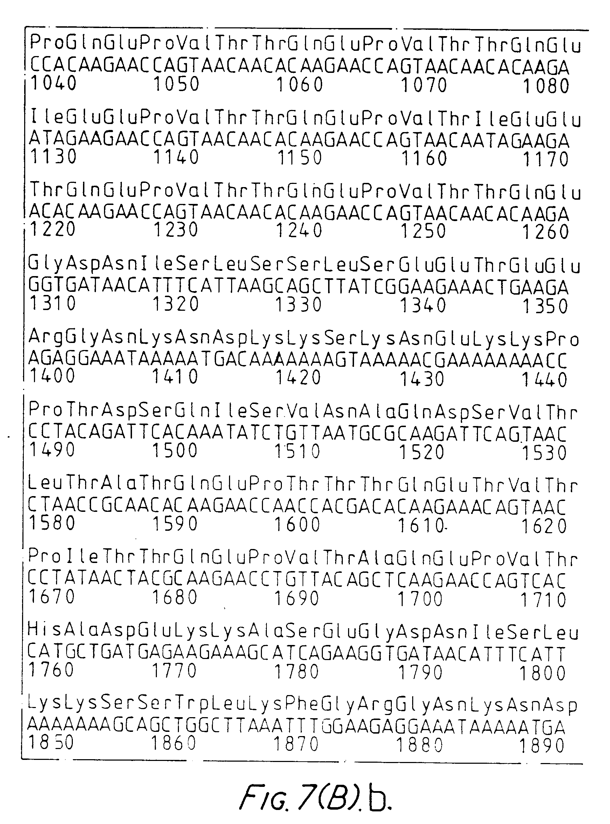

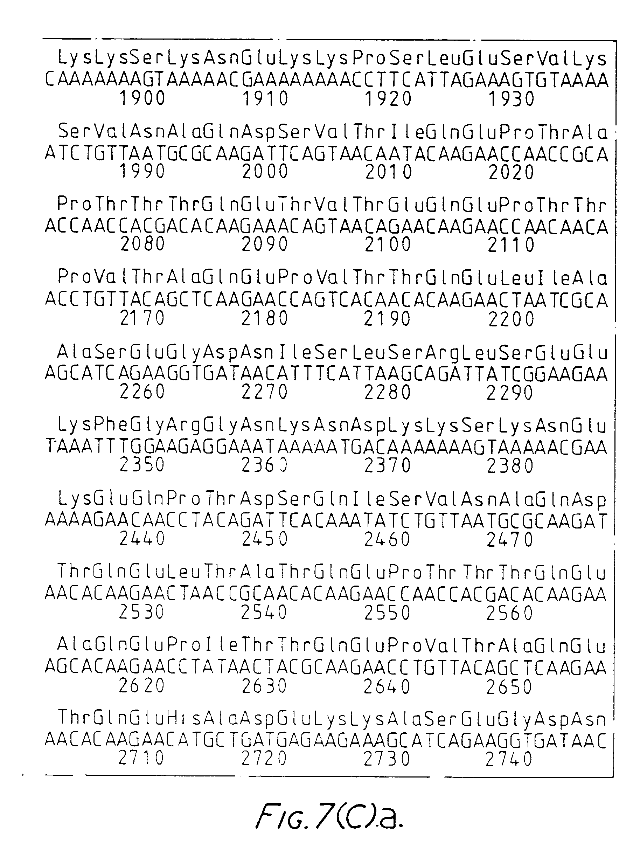

- Figure 6: is a Western blot of ring-stage infected erythrocytes digested with chymotrypsin (20µg/ml) for 0 min. (1), 20 min. (2) and 60 min. (3). Subsequent to enzyme digestion the intact erythrocytes were washed, lysed in electrophoresis sample buffer, electrophoresed on a 10% SDS-polyacrylamide gel and then electrophoretically transferred to nitrocellulose. The nitrocellulose filters were then probed with rabbit anti-RESA at a dilution of 1:500. Molecular weights are indicated in Kdaltons, and correspond to RESA (155Kd), β-galactosidase (116Kd) and phosphorylase-b (93Kd).
- Figure 7: shows the nucleotide sequence and predicted amino acid sequence of the FIRA gene. The nucleotide sequence was determined by the dideoxy procedure (8). The EcoR1 linker ligated to the 3' end during construction of the library was absent and so the sequence is incomplete at the 3' end, perhaps due to a deletion.
- Figure 8: shows the structure of the FIRA gene as deduced from the sequence given in Figure 7.

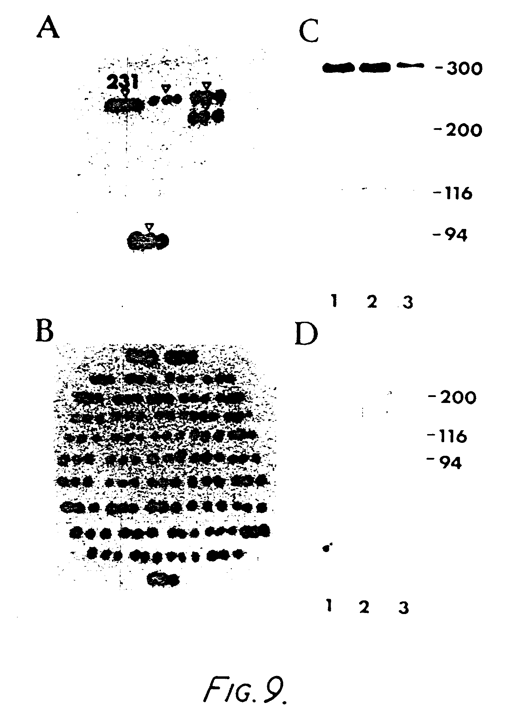

- Figure 9: shows immunoassays (A & B) and Western blots (C & D) with human antibodies affinity-purified from a serum pool derived from individuals exposed to malaria. In A and C the antibodies were purified on a FIRA-Sepharose absorbent whereas in B & D the antibodies were purified on an λamp3-Sepharose absorbent. The P.falciparum isolates in C and D were: 1, FC27 from Papua New Guinea; 2, K1 from Thailand; and 3, NF7 from Ghana.
- Figure 10: shows affinity purified anti-FIRA antibodies assayed by solid-phase ELISA using microtitre plates coated with purified fusion polypeptides (2µg/ml) corresponding to: ○, a fragment of FIRA; □, 5' repeat by RESA; △, 3' repeat of RESA.

### DETAILED DESCRIPTION OF THE INVENTION

### MATERIALS AND METHODS

### P.falciparum isolates

Isolates FCQ27/PNG (FC27), IMR143/PNG (IMR143), IMR144/PNG (IMR144) and MAD71/PNG (MAD71) were obtained through collaboration with the Papua New Guinea Institute of Medical Research. NF7, originating from Ghana, and K1 originating from Thailand were obtained from D.Walliker, Edinburgh University.

### Colony Immunoassays

Replicas of arrays of antigen-positive clones were grown overnight at 30°C, induced at 38°C, and lysed (7). Sera were absorbed to remove anti-E.coli reactivity, diluted 1:500 at pH 9.6 in 3% bovine serum albumin and finally incubated with ¹²⁵I protein A from Staphylococcus aureus and autoradiographed overnight (7).

### Sera

Sera were obtained with informed consent from individuals from Madang, Papua New Guinea. Some patients presented with acute malaria while in others, asymptomatic parasitemia was detected in the course of routine surveys. Parasitemic individuals were treated with chloroquine. Parental consent was obtained before taking samples from children.

### Hybridization experiments

The phage DNA was purified by CsCl-equillbrium density centrifugation, digested with EcoRI, and size-fractionated on a 1% low-melting agarose-gel, recovered by phenol extraction and labelled by nick-translation. 3ml of labelled insert (3 x 10⁵cpm) in 1ml 0.75 M NaCl/0.75 M Na citrate/50% formamide/50µg ml⁻¹ salmon sperm DNA/10µg ml⁻¹ poly (C)/0.02% Ficoll/0.02% polyvinylpyrollidone/0.02% BSA was hybridized to the array of antigen-positive clones. The inserts were subcloned in pUC-9 (9), purified and then nick-translated as described above and used in Southern blot experiments.

### Isolation and sequencing of cloned chromosomal segments

The chromosomal RESA clones were isolated from a λgt10 library, and the EcoR1 inserts subcloned into pUC8. Rsa I, Aha III and Ssp I fragments of the EcoR1 inserts were subcloned into M13mp18 and mp19 vectors, and sequenced by the dideoxy technique (8). Synthetic primers were also used. The results were processed by the program of Staden (10). The sequence shown consists of the 3.5 Kb chromosomal EcoR1 fragment, joined at the EcoR1 site to that of the cDNA clone Ag 46.

The chromosomal FIRA clone was initially identified as a 6 Kb Aha III fragment in λgt10. This Aha III fragment was subcloned into pUC8. Pvu II and Rsa I fragments were then subcloned into M13mp8 and 9 vectors and sequenced by the dideoxy technique.

### Affinity purification of anti-RESA and anti-FIRA antibodies.

Induced cultures (50ml) of clones Ag28, Ag231 and λamp3 were prepared as described previously (5 and 6). The pelleted bacteria were sonicated in 100mM Na phosphate buffer, pH 6.8/10mM dithiothreitol followed by mixing at room temperature with the addition of 1% NaDodSO₄. The soluble bacterial proteins were equilibrated with 100mM Na phosphate, pH 6.8/1mM dithiothreitol/0.1% NaDodSO₄ by passage through Sephadex G-10 and conjugated to CNBr-activated Sepharose (Pharmacia, Sweden) at room temperature according to the manufacturers instructions.

A pool of human sera collected from individuals living in Papua New Guinea was clarified by centrifugation, diluted with an equal volume of phosphate buffered saline (Pi/NaCl) and preabsorbed on a λamp3-Sepharose absorbent before passage over the Ag28 or Ag231 absorbent. Non-specifically bound proteins were removed by repeated wash cycles of 100mM Na borate/500mM NaCl/0.05% Tween 20, pH 8.5 followed by Pi/NaCl. Bound antibodies were eluted with 100mM glycine/150mM NaCl, pH 2.6 and immediately adjusted to pH 7.0 with 2M Tris;HCl, pH 8.0.

### Western blots

Protein extracts of cultures of P.falciparum were prepared and fractionated on 7.5% polyacrylamide/NaDodSO₄ gels. Proteins from the gels were transferred electrophoretically to nitrocellulose, incubated in 5% non-fat milk powder in Pi/NaCl before reaction with affinity purified human antibodies. The filters were incubated with ¹²⁵I-labelled protein A and autoradiographed.

### Immunoelectronmicroscopy

Human antibodies affinity purified on Ag28 and Ag231 immunosorbents, or rabbit antisera raised against the fused polypeptide produced by Ag28 were used in immunoelectronmicroscopy employing the protein A-gold procedure. Samples for immunoelectronmicroscopy were fixed with 0.25% glutaraldehyde (10 min at 25°C), diluted in 50mM NH₄Cl in 0.1M phosphate buffer, pH7.4, and then left in fresh 50mM NH₄Cl in phosphate buffer for 30 min. Cells were then washed twice in phosphate buffer and dehydrated in 70% ethanol before being embedded in L.R. White resin, hard grade (London Resin Co. Ltd., Basingstoke, England). Sections were incubated in 1% bovine serum albumin or ovalbumin in 0.05M phosphate, pH7.4, containing 0.25% Tween-20 (PO₄:Tween) for 5 min. before transfer to a drop of rabbit anti-RESA antiserum (diluted 1:100) or affinity-purified human anti-RESA antibodies in PO₄:Tween for 30-60 min. at room temperature. After being washed in PO₄:Tween the sections were transferred to protein A-gold (E-Y Laboratories, Inc.) diluted 1:10 in PO₄:Tween for 30-60 min. After further washing, the sections were stained with aqueous uranyl acetate. Isolated merozoites were fixed at 4°C in 0.25% glutaraldehyde for 10 min. and then processed in the same manner as infected cells.

### RESULTS - RESA

### Isolation of a RESA cDNA clone from FC27

The preparation of the RESA cDNA clones is described in detail in the Examples of International Patent Specification No. PCT/AU84/00016, and incorporated herein by reference.

### Identification of the RESA polypeptides

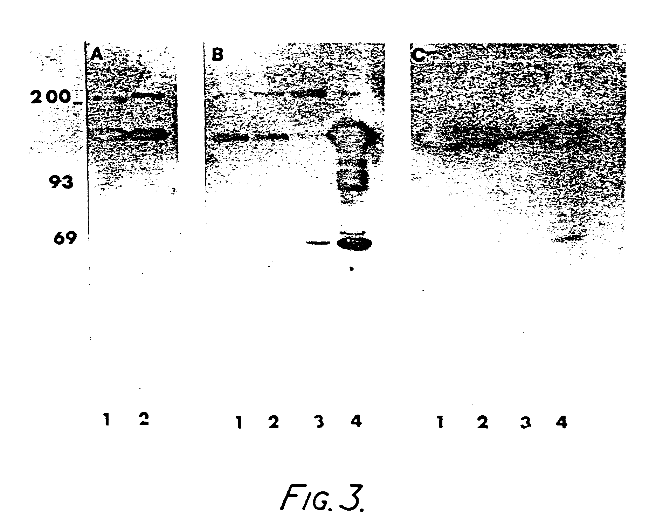

Human antibodies specific for the RESA polypeptides were purified by affinity chromatography. In Western blots the antibodies reacted with a prominent band at Mr 155,000 which, in some experiments, resolved into a closely migrating doublet. A higher molecular weight polypeptide reacting with the anti-RESA antibodies varied in size in different isolates (Figure 3A); it was at Mr 210,000 in isolate FC27. In addition, a smaller molecular weight polypeptide (Mr 80,000) was detected in some antigen preparations (Figure 3A). The abundance of the Mr 210,000 polypeptide was greatest in schizonts (Figure 3B). In contrast, the Mr 155,000 antigen was abundant in the merozoites, rings and trophozoites with small amounts of schizonts (Figure 3, B and C.)

The solubility of RESA in detergents was determined to examine the nature of the interaction between RESA and the erythrocyte membrane. The Mr 210,000 polypeptide was soluble in solutions of the nonionic detergent Triton X-100, as was the most of the Mr 155,000 polypeptide present in merozoites (Figure 3B). In contrast, the bulk of the Mr 155,000 antigen in rings and other life-cycle stages was insoluble in Triton X-100 but could be solubilised in electrophoresis sample buffer containing SDS and 2-mercaptoethanol (Figure 3, B and C).

When identical immunoblots were probed with monoclonal antibodies raised against the Ag28 fused polypeptide, or antisera raised in mice against RESA synthetic peptides, the Mr 210,000 polypeptide was not detected although the Mr 155,000 polypeptide gave a strong signal. Thus, it appears that the Mr 210,000 polypeptide is another gene product that cross-reacts with RESA and not the initial RESA translation product.

### Antibodies against RESA in patients from Africa react with RESA from a Papua New Guinea strain.

Previous studies with mouse antibodies against RESA fused polypeptides expressed in E.coli demonstrated cross reactions with all P.falciparum strains tested, from diverse locations. These RESA cDNA clones were isolated by virtue of their reactivity to sera from Papua New Guinea. To determine whether equivalent antibodies that cross react with RESA from widely differing locations occur in humans exposed to P.falciparum, African sera were tested against a number of cDNA clones expressing portions of RESA, derived from the Papua New Guinea strain FC27. The sera were reacted with an array of 133 independently isolated antigen positive clones, 16 of which encoded RESA, by the in situ colony immunoassay procedure as described (7). Both African sera reacted with the RESA cDNA clones. The extent of reaction was quite comparable to many of the PNG sera. However, it is important to note that the extent of reaction varies considerably in different PNG sera. The African sera also reacted with a variety of other cDNA clones including cDNA clones that encode FIRA that consists largely of divergent repeats of a hexapeptide sequence. In contrast, they did not react with cDNA clones encoding the strain-specific S-antigen of FC27. Thus RESA polypeptides from geographically diverse areas must share non-reacting epitopes that are natural immunogens in man.

### Antigenic determinants of RESA

All RESA cDNA expression clones previously studied immunologically were bounded at the 5' terminus by the internal EcoR1 site. To examine whether any antigenic determinants were located 5' to this site, the large EcoR1 fragment from NF7 AG13 was subcloned into pUC9, randomly fragmented by sonication and the fragments were recloned in λAmp3. To identify clones expressing defined regions of this fragment, the resulting clones were screened by hybridization with 3 different restriction fragments, located 5' to the repeats, spanning the repeats and 3' to the repeats, respectively. Selected clones were then examined for expression of large fused polypeptides, detectable by Coomassie blue staining after polyacrylamide gel electrophoresis of total protein extracts from the cells. Because there are multiple stop codons in all but the correct frame of the sequence, it could be concluded that such clones expressed defined fragments of RESA, 5' to any fragments that had previously been analysed for antibody binding.

Clones expressing 5' repeats were then examined by in situ colony immunoassays with sera from PNG patients with a history of exposure to P.falciparum. Some clones containing the 5' repeat segment reacted with the sera. It is concluded that there are antigenic determinants that are natural immunogens in man in the 5' RESA repeats, as well as the 3' repeats.

A 36 amino acid peptide corresponding to the sequence from residue 17 to residue 52 in exon 1 of RESA (Figure 1) was synthesised and used to test sera from individuals exposed to malaria for antibodies to this region of RESA. Some individuals had significant levels of antibodies reactive with this peptide as measured in a solid-phase radio-immunoassay. Thus there are naturally immunogenic epitopes in exon 1 of RESA which must be encoded by non-repeat sequences.

### Immunogenicity of RESA sequences

RESA/β-galactosidase fused polypeptides were isolated from clones expressing the 3' and 5' repeats of RESA. These proteins were tested for immunogenicity by immunising rabbits with 0.25-0.5mg amounts of antigen together with complete Freund's adjuvant. The rabbits were boosted with similar amounts of antigen in incomplete adjuvant 4-6 weeks later. In each case, antibodies were elicited which reacted with the RESA molecule expressed in P.falciparum growing in vitro.

Three RESA synthetic peptides (Table 1) conjugated to Keyhole Limpet Haemocyanin, were used to immunise mice and the resulting antisera were assayed against each of the three peptides conjugated to bovine serum albumin, and against fused polypeptides corresponding to the 3' and 5' repeats of RESA and sonicates of infected erythrocytes. All mice immunised with these peptides produced antibodies that were reactive with the homologous peptide and the fused polypeptide containing that sequence. In addition, peptide RESA 3'-2 (EENV x4), induced antibodies that also reacted with the other 3' repeat peptide, RESA 3'-1 (EENVEHDA) which has a 5 amino acid sequence in common. The reverse, however, was not true: anti-RESA 3'-1 antibodies did not react with RESA 3'-2.

When these anti-peptide antisera were assayed on peptide-BSA conjugates there was no apparent cross-reactivity between the 5' and 3' repeats of RESA. However, assaying the same sera on fused polypeptides revealed that the peptides had induced antibodies that reacted with both repeat structures, although the reaction with the heterologous repeat was very weak in comparison to that with the homologous repeat.

The anti-peptide antisera were used to probe Western blots of infected erythrocytes. All of the antisera reacted specifically with the Mr 155,000 RESA polypeptide.

**TABLE 1**

| Sequences and synthetic peptides corresponding to repeats in RESA | | |
|---|---|---|
| Region of RESA | Repeat Sequences | Peptides Synthesized* |
| 3' Repeat | EENVEHDA (5)⁺ | RESA 3'-1 EENVEHDA |
| | EENA (1) | |
| | EENV (29) | RESA 3'-2 (EENV)n n ∼4 |
| | EE-V (4) | |
| | EEYD (3) | |
| 5' Repeat | -EENEEEHTV- (1) | |
| | DDEHVEEHT-A (1) | |
| | DDEHVEEPTVA (2) | RESA 5'-1 DDEHVEEPTVAY |
| | -DEHVEEPTVA (1) | |
| | -EEHVEEPTVA (1) | |
| | -EEHVEEP--A (1) | |

| | | |
|---|---|---|
| * The peptides were synthesized by the Merrifield solid-phase method except the RESA 5'-1 peptide was synthesized by the FMOC solid-phase synthesis methodology of Atherton et al (11) on a Kieselguhr KA resin support. | | |
| + The numbers in brackets indicate the number of times the respective sequences occur within the blocks of repeats. | | |

### Location of RESA

RESA was detected by immunoelectronmicroscopy at the membrane of erythrocytes infected with ring-stage parasites but not in association with immature parasites within the erythrocyte (Figure 5). In contrast, the membranes of erythrocytes containing mature parasites were not labelled, but gold particles were associated with electron-dense organelles presumed to be micronemes within the parasite cytoplasm (Figure 4). Control antibodies to S antigens did not react with merozoites or the erythrocyte membrane.

The labelling of merozoites was clearly internal, with no indication of specific labelling of the merozoite surface. Labelling occurred in clusters away from the nucleus and occasionally over a rhoptry. In other merozoites, gold particles were more dispersed but were located near the rhoptries, which were particle-free. Similar distributions of gold were observed with both affinity-purified human antibodies and rabbit antibodies raised against the cloned antigen, although higher background labelling was evident with the affinity-purified human antibodies. The specificity of the observed patterns of labelling was demonstrated by the different patterns, or by the lack of labelling when the same procedures were used with affinity-purified human antibodies or rabbit antisera to other cloned P.falciparum antigens (e.g. S antigen).

The location of RESA was further examined by studying its accessability to attack by proteolytic enzymes. When intact erythrocytes infected with ring-stage parasites (approximately 5% parasitaemia) were treated with chymotrypsin or trypsin, the Mr 155,000 polypeptide was partially cleaved at a limited number of sites generating two main fragments which like the intact molecule reacted with anti-RESA antibodies (Figure 6). This result indicates that at least part of the RESA molecule is exposed on the external surface of the ring-infected erythrocyte.

### Inhibition of parasite growth in vitro

Asynchronous cultures of P.falciparum were cultured for 48 hours in the presence of affinity-purified human anti-RESA antibodies. The degree of inhibition was variable with typical results showing 20-40% inhibition compared with control cultures.

### RESULTS - FIRA

### cDNA clones expressing FIRA

FIRA cDNA clones reacted with up to 93% of a set of more than 100 PNG sera from 65 individuals, varying in clinical status. Further, they gave the most intense signals with a majority of the sera, although many sera reacted strongly with additional clones.

### FIRA is encoded by a single polymorphic gene

Chromosomal DNA from 5 P.falciparum isolates (FC27, IMR143, IMR144, MAD71 from Papua New Guinea, and NF7 from Ghana) was restricted with EcoRI, AhaIII and RsaI and analysed by Southern blotting. In each isolate, a single very large (>20 kb) EcoRI fragment hybridized (data not shown). In the AhaIII and RsaI digests, smaller single fragments of varying sizes hybridized, revealing that the FIRA gene was polymorphic and present in each isolate investigated. The different fragment sizes most likely represent different alleles of the FIRA gene. As at least three different alleles were detected in only 5 different isolates, the total number of alleles is presumably very large. The single fragment size in each isolate is in accord with a haploid genome in blood stage Plasmodium.

### Identification of the FIRA polypeptide

Human antibodies specific for the FIRA polypeptide (Fig. 9) were purified by affinity chromatography. In Western blots the antibodies reacted strongly with a P.falciparum polypeptide of very large apparent size, nominally of Mr >300,000, that was present in each isolate (Fig.9C). Although there were no accurate size markers in this extreme range, the mobility of FIRA was considerably less than that of the Mr 200,000 S antigen of FC27. Isolate differences in the sizes of FIRA polypeptides that might be expected to correlate with the slight differences in size of the DNA fragments could not be detected (Fig. 9C). The antibodies also reacted weakly and variably with a number of smaller polypeptides (Fig. 9C), presumably proteolytic cleavage products of the Mr >300,000 molecule. The control antibodies, purified from the same serum on a vector absorbent did not react (Fig.9D). Further, antibodies purified from the same serum on absorbents from other antigen-positive clones reacted specifically with other polypeptides, not the Mr >300,000 polypeptide (data not shown).

It is concluded that FIRA is a very large polypeptide that is expressed in each isolate of P.falciparum tested, and that antibodies to the allele of FIRA expressed by FC27 cross-react with the alleles expressed by K1 and NF7.

### Location and stage specificity of FIRA and its mRNA

The affinity-purified human antibodies and serum from mice immunized with clone Ag231 or members of the Ag231 family reacted with mature parasites (containing pigment) and also with cells containing immature (ring-form) parasites. The fluorescence over ring-infected cells was uneven and apparently distributed beyond the limits of the parasite. Hence it is likely that FIRA is external to the parasite, although no staining of the erythrocyte surface was detected when the antibodies were reacted in suspension with non-fixed parasitized cells or with lightly glutaraldehyde-fixed and air-dried monolayers of parasitized erythrocytes (12).

The stage specificity of FIRA is therefore in some ways analogous to RESA (2). Hybridization of cDNA prepared from mRNA of highly purified merozoites to the array of 133 colonies revealed another parallel with RESA. All members of the Ag231 family hybridized to merozoite cDNA. Remarkably, the only other clones in this array or in a separate array of 78 antigen positive clones, that hybridize to merozoite cDNA encode RESA (2,13). Hence FIRA and RESA mRNAs are unusual among mRNAs for P.falciparum antigens in that they are greatly enriched in merozoites.

### FIRA Sequence

The chromosomal clone encompassing the AhaIII fragment, cloned in λgt10 and designated Ag231.5 has been fully sequenced. The gene contains an intervening sequence and is remarkably like RESA in overall structure. Exon 1 consists of a segment that may be a signal peptide (although it is very short), then a region of hydrophilic amino acid followed by a stretch of 32 uncharged amino acids. The intervening sequence is located immediately adjacent to this relatively hydrophobic segment. The remaining sequence is composed of blocks of repetitive and interspersed non-repetitive sequences. In all cases, the repetitive sequences occur as groups of 13 hexamers, but the most 5' group of these lack interspersed non-repetitive sequences - i.e. there is a block of 39 hexamers. It appears that a deletion at the 3' end has altered the linker - Aha join, so the structure at the 3' end is uncertain.

### Cross-reactions amongst repeats

Human antibodies affinity-purified on Ag231.6 (FIRA) when tested in an ELISA gave a very strong signal on Ag231.6, a weaker but very definite signal on Ag13.1.7.5 (RESA 5' repeat), and no signal on Ag13 (RESA 3' repeat) (Figure 10). This cross-reaction is consistent with the sequence homology between the repeats in these otherwise distinct antigens.

A full description of the preparation of recombinant DNA molecules, and of recombinant DNA cloning vehicles and vectors, of host cell-cloning vehicle combinations, and of the expression of polypeptides by host cells is contained in International Patent Specification No. PCT/AU84/00016. This specification also describes in detail the use of DNA molecules and polypeptides expressed thereby in serological diagnosis, and in the preparation of single and multivalent vaccines for stimulating protective antibodies against Plasmodia. That description is equally applicable to the present invention and is incorporated herein by reference.

### REFERENCES

1. Kemp, D.J., Coppel, R.L., Cowman, A.F., Saint, R.B., Brown, G.V. & Anders, R.F. (1983) Proc.Natl.Acad.Sci.USA 80, 3787-3791.
2. Coppel, R.L., Cowman, A.F., Anders, R.F., Bianco, A.E., Saint, R.B., Lingelbach, K.R., Kemp, D.J. & Brown, G.V. (1984) Nature (London) 310, 789-792.
3. Ozaki, L.S., Svec, P., Nussenzweig, R.S., Nussenzweig, V. & Godson, G.N. (1983). Cell 34, 815-822.
4. Dame, J.B., Williams, J.L., McCutchan, T.F., Weber, J.L., Wirtz, R.A., Hockmeyer, W.T., Sanders, G.S., Reddy, E.P., Maloy, W.L., Haynes, J.D., Schneider, I., Roberts, D., Diggs, C.L.& Miller, L.H. (1984) Science 225, 593-599.
5. Coppel, R.L., Cowman, A.F., Lingelbach, K.R., Brown, G.V., Saint, R.B., Kemp, D.J. & Anders, R.F. (1983) Nature (London) 306, 751-756.
6. Stahl, H-D., Crewther, P.E., Anders, R.F., Brown, G.V., Coppel, R.L., Bianco, A.E., Mitchell, G.F. and Kemp, D.J. (1985). Proc.Natl.Acad.Sci.USA. 82, 543-547.
7. Stahl, H-D., Coppel, R.L., Brown, G.V., Saint, R.B., Lingelbach, K., Cowman, A.F., Anders, R.F. & Kemp, D.J. (1984) Proc.Nat.Acad.Sci.USA 81, 2456-2460.
8. Sanger, R., Nicklen, S. & Coulson, A.R. (1977) Proc.Nat.Acad.Sci.USA 74, 5463-5467.
9. Messing, J. & Vieira, J. (1982) Gene 19, 269-276.
10. Staden, R. (1980) Nucl.Acids.Res. 8, 3673-3694.
11. Atherton, E., Caviezel, M., Fox, H., Harkiss, D., Over, H., Sheppard, R.C. (1983) J.Chem.Soc.Perkin Trans. 1, 65-73.
12. Perlmann, H., Berzins, K., Wahlgren, M., Carlsson, J., Björkmann, A., Patarvoyo, M.E., and Perlmann, P. (1984) J.Exp.Med. 159, 1686-1704.
13. Anders, R.F., Coppel, R.L., Brown, G.V., Saint, R.B., Cowman, A.F., Lingelbach, K.R., Mitchell, G.F. and Kemp, D.J. (1984) Molec.Biol.Med. 2, 177-191.

## Claims

1. A DNA molecule comprising a nucleotide sequence coding for an immunogenic polypeptide of P.falciparum, characterised in that said nucleotide sequence substantially corresponds to all or a portion of the base sequence as shown in Figure 7 coding for the Falciparum Interspersed Repeat Antigen (FIRA); or substantially corresponds to all of the base sequence as shown in Figure 1 coding for the Ring-Infected Erythrocyte Surface Antigen (RESA) or a portion thereof other than a portion consisting solely of the sequence 3619-3996 of Figure 1 or portions thereof; or to a functional equivalent of any of the foregoing nucleotide sequences.

2. A DNA molecule as claimed in claim 1 comprising a nucleotide subsequence encoding an amino sequence selected from EENVEHDA, EENA, EENV, EEV, EEYD, EENEEHTV, DDEHVEETA, DDEHVEEPTVA, DEHVEEPTVA, EEHVEEPTVA and EEHVEEPA.

3. A DNA molecule as claimed in claim 2 having a said subsequence other than in said sequence 3619-3996.

4. A DNA molecule as claimed in any one of claims 1 to 3 comprising in its nucleotide sequence a subsequence coding for an antigenic epitope outside the said sequence 3619-3996.

5. A DNA molecule comprising a portion of the nucleotide sequence of Figure 1 or a variant thereof such that upon expression of said DNA molecule an immunogenic polypeptide selected from EENVEHDA, EENA, EENV, EEV, EEYD, EENEEHTV, DDEHVEETA, DDEHVEEPTVA, DEHVEEPTVA, DEHVEEPTVA, EEHVEEPTVA and EEHVEEPA is obtained.

6. A recombinant DNA molecule comprising a nucleotide sequence according to any one of claims 1 to 5, operatively linked to an expression control sequence.

7. A recombinant DNA cloning vehicle or vector capable of expressing all or a portion of at least one polypeptide or protein of P.falciparum, and having inserted therein a nucleotide sequence according to any one of claims 1 to 5, said sequence being operatively linked to an expression control sequence.

8. A recombinant DNA cloning vehicle or vector according to claim 7, characterised in that said nucleotide sequence and said expression control sequence are inserted into a bacteriophage.

9. A recombinant DNA cloning vehicle or vector according to claim 8, characterised in that said bacteriophage is bacteriophage λ Amp 3.

10. A host cell containing a recombinant DNA molecule according to claim 6, or a recombinant DNA cloning vehicle or vector according to claim 7.

11. A synthetic peptide or polypeptide comprising an amino acid sequence displaying the antigenicity of all or a portion of an immunogenic polypeptide of P.falciparum, characterised in that said amino acid sequence substantially corresponds to the amino acid sequence of the FIRA antigen as shown in Figure 7 or an immunogenic portion thereof or substantially corresponds to the amino acid sequence of the RESA antigen as shown in Figure 1 or an immunogenic portion thereof other than a portion encoded by the sequence 3619-3996 of Figure 1 or portions thereof; or to a functional equivalent of any of the foregoing amino acid sequences.

12. A synthetic peptide or polypeptide displaying antigenicity of an immunogenic polypeptide of P.falciparum selected from EENVEHDA, EENA, EENV, EEV, EEYD, EENEEHTV, DDEHVEETA, DDEHVEEPTVA, DEHVEEPTVA, EEHVEEPTVA and EEHVEEPA.

13. A fused polypeptide comprising a polypeptide sequence displaying the antigenicity of an immunogenic polypeptide of P.falciparum as defined in claim 11 or claim 12 and an additional polypeptide as the N-terminal sequence fused thereto.

14. A fused polypeptide according to claim 13, wherein the additional polypeptide is a polypeptide coded for by the DNA of a recombinant DNA cloning vehicle or vector.

15. A composition for stimulating immune responses against P.falciparum antigens in a mammal, comprising at least one immunogenic polypeptide encoded by a nucleotide sequence as defined in any one of claims 1 to 5 together with a pharmaceutically acceptable carrier therefor.

16. A composition according to claim 15, further comprising an adjuvant.

17. A composition for stimulating immune responses against P.falciparum antigens in a mammal, comprising a virus or microorganism in association with a pharmaceutically acceptable carrier, the virus or microorganism having inserted therein a DNA molecule comprising a nucleotide sequence coding for an immunogenic polypeptide of P.falciparum as defined in any one of claims 1 to 5.

18. Use of an immunogenic polypeptide of P.falciparum encoded by a nucleotide sequence as defined in any one of claims 1 to 5 for the preparation of a composition for stimulating immune responses in a mammal against one or more P.falciparum antigens.

## Patentansprüche

1. DNA-Molekül, umfassend eine für ein immunogenes Polypeptid von P.falciparum codierende Nukleotidsequenz, dadurch gekennzeichnet, daß diese Nukleotidsequenz im wesentlichen der gesamten in Figur 7 dargestellten, für das "Falciparum Interspersed Repeat Antigen" (FIRA) codierenden Basensequenz oder einem Teil davon entspricht, oder daß sie im wesentlichen der gesamten in Figur 1 dargestellten, für das "Ring-Infected Erythrocyte Surface Antigen" (RESA) codierenden Basensequenz oder einem Teil davon mit Ausnahme eines solchen Teiles, der ausschließlich aus der Sequenz 3619-3996 der Figur 1 oder Teilen davon besteht, oder einem funktionellen Äquivalent einer der voranstehenden Nukleotidsequenzen entspricht.

2. DNA-Molekül nach Anspruch 1, umfassend eine Nukleotidteilsequenz, die für eine unter EENVEHDA, EENA, EENV, EEV, EEYD, EENEEHTV, DDEHVEETA, DDEHVEEPTVA, DEHVEEPTVA, EEHVEEPTVA und EEHVEEPA ausgewählte Aminosauresequenz codiert.

3. DNA-Molekül nach Anspruch 2 mit einer solchen Teilsequenz, jedoch nicht in der genannten Sequenz 3619-3996.

4. DNA-Molekül nach einem der Ansprüche 1 bis 3, umfassend in seiner Nukleotidsequenz eine Teilsequenz, die für ein antigenes Epitop außerhalb der Sequenz 3619-3996 codiert.

5. DNA-Molekül, umfassend einen Teil der Nukleotidsequenz der Figur 1 oder eine Variante davon derart, daß nach Expression des DNA-Moleküls ein unter EENVEHDA, EENA, EENV, EEV, EEYD, EENEEHTV, DDEHVEETA, DDEHVEEPTVA, DEHVEEPTVA, DEHVEEPTVA, EEHVEEPTVA und EEHVEEPA ausgewähltes immunogenes Polypeptid erhalten wird.

6. Rekombinantes DNA-Molekül, umfassend eine Nukleotidsequenz nach einem der Ansprüche 1 bis 5, welche operativ mit einer Expressionskontrollsequenz verknüpft ist.

7. Rekombinantes DNA-Klonierungsvehikel oder rekombinanter DNA-Klonierungsvektor, der mindestens ein vollständiges Polypeptid oder Protein von P. falciparum oder einen Teil davon exprimieren kann und eine Nukleotidsequenz nach einem der Ansprüche 1 bis 5 insertiert enthält, wobei diese Sequenz operativ mit einer Expressionskontrollsequenz verknüpft ist.

8. Rekombinantes DNA-Klonierungsvehikel oder rekombinanter DNA-Klonierungsvektor nach Anspruch 7, dadurch gekennzeichnet, daß die Nukleotidsequenz und die Expressionskontrollsequenz in einen Bakteriophagen insertiert sind.

9. Rekombinantes DNA-Klonierungsvehikel oder rekombinanter DNA-Klonierungsvektor nach Anspruch 8, dadurch gekennzeichnet, daß der Bakteriophage λ Amp 3 ist.

10. Wirtszelle, enthaltend ein rekombinantes DNA-Molekül nach Anspruch 6 oder ein rekombinantes DNA-Klonierungsvehikel oder einen rekombinanten DNA-Klonierungsvektor nach Anspruch 7.

11. Synthetisches Peptid oder Polypeptid, umfassend eine Aminosäuresequenz, welche die Antigenität des gesamten immunogenen Polypeptids von P. falciparum oder eines Teils davon zeigt, dadurch gekennzeichnet, daß diese Aminosäuresequenz im wesentlichen der Aminosäuresequenz des FIRA-Antigens, wie in Figur 7 dargestellt, oder einem immunogenen Teil davon entspricht oder im wesentlichen der Aminosäuresequenz des RESA-Antigens, wie in Figur 1 dargestellt, oder einem immunogenen Teil davon, der sich von einem durch die Sequenz 3619-3996 der Figur 1 oder Teilen davon codierten Teil unterscheidet, oder einem funktionellen Äquivalent einer der voranstehenden Aminosäuresequenzen entspricht.

12. Synthetisches Peptid oder Polypeptid, welches die Antigenität eines immunogenen Polypeptids von P.falciparum zeigt, ausgewählt unter EENVEHDA, EENA, EENV, EEV, EEYD, EENEEHTV, DDEHVEETA, DDEHVEEPTVA, DEHVEEPTVA, EEHVEEPTVA und EEHVEEPA.

13. Fusioniertes Polypeptid, umfassend eine Polypeptidsequenz, die die Antigenität eines immunogenen Polypeptids von P.falciparum, wie in Anspruch 11 oder Anspruch 12 definiert, aufweist, sowie ein weiteres Polypeptid als daran fusionierte, N-terminale Sequenz.

14. Fusioniertes Polypeptid nach Anspruch 13, worin das zusätzliche Polypeptid ein Polypeptid ist, welches durch die DNA eines rekombinanten DNA-Klonierungsvehikels oder eines solchen Vektors codiert wird.

15. Zusammensetzung zur Stimulierung von Immunantworten gegen P.falciparum-Antigene in einem Säuger, enthaltend mindestens ein immunogenes Polypeptid, welches von einer in einem der Ansprüche 1 bis 5 definierten Nukleotidsequenz codiert wird, zusammen mit einem pharmazeutisch annehmbaren Träger hierfür.

16. Zusammensetzung nach Anspruch 15, welche zusätzlich ein Adjuvans enthält.

17. Zusammensetzung zum Stimulieren einer Immunantwort gegen P.falciparum-Antigene in einem Säuger, enthaltend ein Virus oder einen Mikroorganismus zusammen mit einem pharmazeutisch annehmbaren Träger, wobei das Virus oder der Mikroorganismus ein DNA-Molekül insertiert enthält, welches eine für ein immunogenes Polypeptid von P.falciparum codierende Nukleotidsequenz wie in einem der Ansprüche 1 bis 5 definiert, umfaßt.

18. Verwendung eines immunogenen Polypeptids von P.falciparum, welches von einer wie in einem der Ansprüche 1 bis 5 definierten Nukleotidsequenz codiert wird, für die Herstellung einer Zusammensetzung zum Stimulieren von Immunantworten in einem Säuger gegen ein oder mehrere P.falciparum-Antigen(e).

## Revendications

1. Molécule d'ADN comprenant une séquence nucléotidique codant pour un polypeptide immunogène de P. falciparum, caractérisée en ce que la séquence nucléotidique correspond sensiblement à tout ou à une partie de la séquence de bases telle que représentée sur la figure 7, qui code pour l'antigène de répétition intercalé de Falciparum (FIRA), ou correspond sensiblement à toute la séquence de bases, telle que représentée sur la figure 1, qui code pour l'antiqène de surface d'érythrocyte à noyau infecté (RESA), ou à une partie de celle-ci autre qu'une partie consistant seulement en la séquence 3619-3996 de la figure 1 ou en des parties de celle-ci, ou à un équivalent fonctionnel d'une quelconque des séquences nucléotidiques précédentes.

2. Molécule d'ADN suivant la revendication 1, comprenant une sous-séquence nucléotidique codant pour une séquence amino choisie parmi EENVEHDA, EENA, EENV, EEV, EEYD, EENEEHTV, DDEHVEETA, DDEHVEEPTVA, DEHVEEPTVA, EEHVEEPTVA et EEHVEEPA.

3. Molécule d'ADN suivant la revendication 2, comportant une sous-séquence susdite autre que dans ladite séquence 3619-3996.

4. Molécule d'ADN suivant l'une quelconque des revendications 1 à 3, comprenant dans sa séquence nucléotidique une sous-séquence codant pour un épitope antigène en dehors de ladite séquence 3619-3996.

5. Molécule d'ADN comprenant une partie de la séquence nucléotidique suivant la figure 1 ou une variante de celle-ci telle qu'après expression de ladite molécule d'ADN, un polypeptide immunogène choisi parmi EENVEHDA, EENA, EENV, EEV, EEYD, EENEEHTV, DDEHVEETA, DDEHVEEPTVA, DEHVEEPTVA, EEHVEEPTVA et EEHVEEPA soit obtenu.

6. Molécule d'ADN recombinant comprenant une séquence nucléotidique suivant l'une quelconque des revendications 1 à 5, fonctionnellement reliée à une séquence de contrôle d'expression.

7. Véhicule ou vecteur de clonage d'ADN recombinant, capable d'exprimer tout ou une partie d'au moins un polypeptide ou protéine de P. falciparum et comportant, insérée en lui, une séquence nucléotidique suivant l'une quelconque des revendications 1 à 5, cette séquence étant fonctionnellement reliée à une séquence de contrôle d'expression.

8. Véhicule ou vecteur de clonage d'ADN recombinant suivant la revendication 7, caractérisé en ce que la séquence nucléotidique et la séquence de contrôle d'expression sont insérées dans un bactériophage.

9. Véhicule ou vecteur de clonage d'ADN recombinant suivant la revendication 8, caractérisé en ce que le bactériophage est un bactériophage λ Amp 3.

10. Cellule hôte contenant une molécule d'ADN recombinant suivant la revendication 6, ou un véhicule ou vecteur de clonage d'ADN recombinant suivant la revendication 7.

11. Peptide ou polypeptide synthétique comprenant une séquence d'aminoacides déployant l'antigénicité de tout ou d'une partie d'un polypeptide immunogène de P. falciparum, caractérisé en ce que la séquence d'aminoacides correspond sensiblement à la séquence d'aminoacides de l'antigène FIRA, telle que représentée sur la figure 7, ou à une partie immunogène de celle-ci ou correspond sensiblement à la séquence d'aminoacides de l'antigène RESA, telle que représentée sur la figure 1, ou à une partie immunogène de celle-ci autre qu'une partie codée par la séquence 3619-3996 de la figure 1 ou des parties de celle-ci, ou à un équivalent fonctionnel d'une quelconque des séquences d'aminoacides précédentes.

12. Peptide ou polypeptide synthétique déployant l'antigénicité d'un polypeptide immunogène de P. falciparum choisi parmi EENVEHDA, EENA, EENV, EEV, EEYD, EENEEHTV, DDEHVEETA, DDEHVEEPTVA, DEHVEEPTVA, EEHVEEPTVA et EEHVEEPA.

13. Polypeptide fusionné comprenant une séquence polypeptidique déployant l'antigénicité d'un polypeptide immunogène de P. falciparum suivant la revendication 11 ou la revendication 12 et un polypeptide supplémentaire, en tant que séquence à N terminal fusionnée à lui.

14. Polypeptide fusionné suivant la revendication 13, dans lequel le polypeptide supplémentaire est un polypeptide codé par l'ADN d'un véhicule ou vecteur de clonage d'ADN recombinant.

15. Composition de stimulation de réponses immunitaires contre des antigènes de P. falciparum chez un mammifère, comprenant au moins un polypeptide immunogène codé par une séquence nucléotidique suivant l'une quelconque des revendications 1 à 5 conjointement à un support pharmaceutiquement acceptable.

16. Composition suivant la revendication 15, comprenant en outre un adjuvant.

17. Composition de stimulation de réponses immunitaires contre des antigènes de P. falciparum chez un mammifère, comprenant un virus ou micro-organisme en association avec un support pharmaceutiquement acceptable, le virus ou micro-organisme ayant inséré en lui une molécule d'ADN comprenant une séquence nucléotidique codant pour un polypeptide immunogène de P. falciparum, suivant l'une quelconque des revendications 1 à 5.

18. Utilisation d'un polypeptide immunogène de P. falciparum codé par une séquence nucléotidique suivant l'une quelconque des revendications 1 à 5, pour la préparation d'une composition de stimulation de réponses immunitaires chez un mammifère à l'encontre d'un ou plusieurs antigènes de P. falciparum.
